# EUROPEAN PATENT SPECIFICATION

(11) **EP 3 444 236 B1**
(45) Date of publication and mention of the grant of the patent: **30.12.2020**
(21) Application number: 17782388.7
(22) Date of filing: 11.04.2017
(51) Int. Cl.: C07C 265/14, C07C 265/08, C08G 18/71, C08G 18/76, G02B 1/04

(54) **XYLYLENEDIISOCYANATE COMPOSITION, RESIN, AND POLYMERIZABLE COMPOSITION**
XYLYLENDIISOCYANATZUSAMMENSETZUNG, HARZ UND POLYMERISIERBARE ZUSAMMENSETZUNG
COMPOSITION DE XYLYLÈNE DIISOCYANATE, RÉSINE ET COMPOSITION POLYMÉRISABLE

(30) Priority: 11.04.2016 JP 2016078597; 06.01.2017 JP 2017001099
(43) Date of publication of application: 20.02.2019
(73) Proprietor: Mitsui Chemicals, Inc., Minato-ku Tokyo 105-7122 (JP)
(72) Inventor: KUMA, Shigetoshi, Omuta-shi Fukuoka 836-8610 (JP)
(74) Representative: Hoffmann Eitle
(86) International application number: PCT/JP2017/014828
(87) International publication number: WO 2017/179575

(56) References cited:
- EP-A1- 3 470 393
- WO-A1-2015/155366
- JP-A- H0 578 304
- JP-A- 2008 523 128
- JP-B1- S4 517 434
- US-A- 3 549 504
- US-A- 5 302 749

## Description

### TECHNICAL FIELD

The present invention relates to a xylylene diisocyanate composition, the use of the xylylene diisocyanate composition, and a resin and a polymerizable composition using the xylylene diisocyanate composition.

### BACKGROUND ART

As an alternative of a glass lens, a plastic lens has been recently widespread.

As the plastic lens, a poly(thio)urethane lens obtained by reaction of a polyisocyanate and a polyol component and/or a polythiol component has been known.

It has been known that the polyisocyanate that is a material of the poly(thio)urethane lens is obtained by reaction of amine with phosgene, and a chlorine compound is produced as a byproduct at the time of the reaction (ref: for example, Patent Document 1).

Patent Document 1 discloses a xylylene diisocyanate containing a chloromethylbenzyl isocyanate at a ratio of 0.1 weight% as the chlorine compound that is produced as a byproduct.

US 5,302,749 describes a method for stabilizing an isocyanate compound by adding 10 to 5000 ppm of phenol to the isocyanate compound. In an example, a m-xylylene diisocyanate composition having a hydrolyzable chlorine content of 0.01% (= 100 ppm) is mentioned.

US 3,549,504 is concerned with the purification of polyisocyanates such as xylylene diisocyanate, which are obtained by phosgenation. In an example, fractional distillation afforded a xylylene diisocyanate composition having a hydrolyzable chlorine content of 100 ppm.

In WO 2015/155366 A1, a composition for producing transparent polythiourethane bodies is described, which contains or consists of A) a polyisocyanate component containing at least one polyisocyanate with a functionality of isocyanate groups of at least 2 per molecule, B) a thiol component containing at least one polythiol with a functionality of thiol groups of at least two per molecule. According to an example, fractional distillation yielded a xylylene diisocyanate composition having a hydrolyzable chlorine content of 105 ppm.

### Citation List

### Patent Document

Patent Document 1: International Publication No. WO2007/010996A1

### SUMMARY OF THE INVENTION

### PROBLEM TO BE SOLVED BY THE INVENTION

However, in an optical material, further improvement of the yellowing resistance is required.

The present invention provides a xylylene diisocyanate composition and a polymerizable composition for producing a resin having excellent yellowing resistance.

### MEANS FOR SOLVING THE PR OBLEM

The present invention [1] includes a xylylene diisocyanate composition containing a xylylene diisocyanate and a chloromethylbenzyl isocyanate, wherein the content ratio of the chloromethylbenzyl isocyanate is 10 mass ppm or more and 500 mass ppm or less.

The present invention [2] includes the use of the xylylene diisocyanate composition described in the above [1] in the production of an optical material.

The present invention [3] includes a resin that is a reaction product of the xylylene diisocyanate composition described in the above [1] and a polyol component and/or a polythiol component.

The present invention [4] includes the resin described in the above [3], wherein it is an optical material.

The present invention [5] includes the resin described in the above [4], wherein it is an optical lens.

The present invention [6] includes a polymerizable composition containing the xylylene diisocyanate composition described in the above [1] and a polyol component and/or a polythiol component.

### EFFECT OF THE INVENTION

A xylylene diisocyanate composition of the present invention contains a xylylene diisocyanate and a chloromethylbenzyl isocyanate, and the content ratio of the chloromethylbenzyl isocyanate is 10 mass ppm or more and 500 mass ppm or less.

Thus, a resin obtained from the xylylene diisocyanate composition and a polymerizable composition of the present invention has excellent yellowing resistance and excellent production efficiency.

Furthermore, the resin obtained from the xylylene diisocyanate composition and the polymerizable composition of the present invention is capable of retaining its optical properties and improving the dyeability.

### DESCRIPTION OF EMBODIMENTS

A xylylene diisocyanate composition of the present invention contains a xylylene diisocyanate and a chloromethylbenzyl isocyanate.

Examples of a structural isomer of the xylylene diisocyanate (bis(isocyanatomethyl)benzene) include 1,2-xylylene diisocyanate (o-xylylene diisocyanate (o-XDI)), 1,3-xylylene diisocyanate (m-xylylene diisocyanate (m-XDI)), and 1,4-xylylene diisocyanate (p-xylylene diisocyanate (p-XDI)).

These xylylene diisocyanates may be contained alone or in combination of two or more.

As the xylylene diisocyanate, preferably, a 1,3-xylylene diisocyanate and a 1,4-xylylene diisocyanate are used, more preferably, a 1,3-xylylene diisocyanate is used.

The content ratio (purity) of the xylylene diisocyanate with respect to the total amount of the xylylene diisocyanate composition is, for example, 99.90 mass% or more, preferably 99.95 mass% or more, and for example, 99.999 mass% or less, preferably 99.990 mass% or less.

The content ratio of the xylylene diisocyanate can be measured in conformity with Examples to be described later.

The chloromethylbenzyl isocyanate is a chlorine compound that is produced as a byproduct at the time of the production of the xylylene diisocyanate by a production method of a xylylene diisocyanate composition to be described later.

Examples of a structural isomer of the chloromethylbenzyl isocyanate include orthochloromethylbenzyl isocyanate, methachloromethylbenzyl isocyanate, and parachloromethylbenzyl isocyanate.

These chloromethylbenzyl isocyanates may be contained alone or in combination of two or more.

As the chloromethylbenzyl isocyanate, preferably, a methachloromethylbenzyl isocyanate and a parachloromethylbenzyl isocyanate are used, more preferably, a methachloromethylbenzyl isocyanate is used, and when the xylylene diisocyanate is produced, a chloromethylbenzyl isocyanate corresponding to each of the above-described structural isomers of the xylylene diisocyanate is produced as a byproduct.

The content ratio of the chloromethylbenzyl isocyanate with respect to the total amount of the xylylene diisocyanate composition is 10 mass ppm or more, preferably 50 mass ppm or more, more preferably 100 mass ppm or more, and 500 mass ppm or less, preferably 400 mass ppm or less, more preferably 300 mass ppm or less.

The content ratio of the chloromethylbenzyl isocyanate can be measured by analysis with a gas chromatograph in conformity with Examples to be described later.

When the content ratio of the chloromethylbenzyl isocyanate is the above-described lower limit or more, a resin can be stably produced from the xylylene diisocyanate composition. When the content ratio of the chloromethylbenzyl isocyanate is the above-described upper limit or less, a resin having improved yellowing resistance (described later) can be obtained.

In view of improvement of the dyeability of the resin (described later), the content ratio of the chloromethylbenzyl isocyanate with respect to the total amount of the xylylene diisocyanate composition is 10 mass ppm or more, preferably 30 mass ppm or more, and 500 mass ppm or less, preferably 300 mass ppm or less, more preferably below 200 mass ppm, further more preferably 100 mass ppm or less.

The xylylene diisocyanate composition can be, for example, obtained by subjecting a xylylene diamine that is a material to isocyanate reaction.

Examples of a structural isomer of the xylylene diamine that is a material (hereinafter, simply referred to as a xylylene diamine) include 1,2-xylylene diamine (o-xylylene diamine (o-XDA)), 1,3-xylylene diamine (m-xylylene diamine (m-XDA)), and 1,4-xylylene diamine (p-xylylene diamine (p-XDA)).

As a method for subjecting the xylylene diamine to isocyanate reaction, a phosgenation method is used.

To be specific, examples of the phosgenation method include a method in which the xylylene diamine directly reacts with phosgene (hereinafter, may be referred to as a cooling/heating two-stage phosgenation method) and a method in which a hydrochloride obtained by reaction of the xylylene diamine with a hydrochloric acid (hydrogen chloride) reacts with the phosgene in an inert solvent (described later) (hereinafter, may be referred to as a phosgenation method of amine hydrochloride). Preferably, a phosgenation method of amine hydrochloride is used.

The cooling/heating two-stage phosgenation method is made up of a cooling phosgenation reaction and a heating phosgenation reaction. The main reaction of the cooling phosgenation reaction is the production of a carbamylchloride and an amine hydrochloride, and the main reaction of the heating phosgenation reaction is thermal decomposition of the carbamylchloride to an isocyanate and phosgenation of the amine hydrochloride to an isocyanate.

The embodiment of the reaction is not particularly limited, and generally, a reaction vessel in which the inside thereof is capable of being sufficiently stirred and equipped with a phosgene introduction tube is used.

In the cooling phosgenation reaction, the reaction vessel is charged with an inert solvent, and the pressure of the inside thereof is, for example, a normal pressure or more, and for example, 1.0 MPa (gauge pressure) or less, preferably 0.5 MPa (gauge pressure) or less; the temperature thereof is cooled to, for example, 0°C or more, and for example, 80°C or less, preferably 60°C or less; and the phosgene of, for example, 1 time or more, and for example, 10 times or less, preferably 6 times or less of the stoichiometry of the above-described xylylene diamine is introduced, so that the above-described xylylene diamine dissolved in the inert solvent is added. Meanwhile, the reaction liquid is retained in a range of, for example, 0°C or more, and for example, 80°C or less, preferably 60°C or less, and the produced hydrogen chloride is emitted to the outside of the reaction vessel through a reflux condenser.

Examples of the inert solvent include aromatic hydrocarbons such as benzene, toluene, xylene, and ethyl benzene; fatty acid esters such as ethyl acetate, butyl acetate, and amyl acetate; aromatic carboxylates such as methyl salicylate, dimethyl phthalate, dibutyl phthalate, and methyl benzoate; chlorinated aromatic hydrocarbons such as monochlorobenzene, orthodichlorobenzene, paradichlorobenzene, and trichlorobenzene; and chlorinated hydrocarbons such as chloroform and carbon tetrachloride.

These inert solvents can be used alone or in combination of two or more. Furthermore, after the reaction with the phosgene, these inert solvents can be retrieved, refined by, for example, distillation, and reused.

As the inert solvent, preferably, chlorinated aromatic hydrocarbons are used, more preferably, orthodichlorobenzene is used.

The mixing amount (total amount) of the inert solvent with respect to 100 parts by mass of the xylylene diamine is, for example, 400 parts by mass or more, preferably 500 parts by mass or more, and for example, 3000 parts by mass or less, preferably 2000 parts by mass or less.

Next, in the heating phosgenation reaction, the pressure of the inside of the reaction vessel is, for example, a normal pressure or more, preferably 0.05 MPa (gauge pressure) or more, and for example, 1.0 MPa (gauge pressure) or less, preferably 0.5 MPa (gauge pressure) or less, and the temperature thereof is increased in a range of, for example, 80°C or more, and for example, 180°C or less for, for example, 30 minutes or more and for example, 5 hours or less. After the increase in temperature, the reaction continues for, for example, 30 minutes or more, and for example, 8 hours or less, and the reaction terminates when the reaction liquid (slurry) is completely dissolved. At the time of the increase in temperature and the high temperature reaction, the dissolved phosgene is vaporized and emitted to the outside of the reaction vessel through the reflux condenser, so that the phosgene is appropriately introduced until the reflux amount from the reflux condenser can be confirmed. After the termination of the heating phosgenation reaction, an inert gas such as nitrogen gas is introduced into the reaction vessel within a range of, for example, 80°C or more, and for example, 180°C or less, and the excessive phosgene and the excessive hydrogen chloride that are dissolved are purged.

In the phosgenation method of the amine hydrochloride, first, the hydrochloride of the xylylene diamine is synthesized.

To be specific, for example, a reaction vessel in which stirring can be performed and equipped with a hydrogen chloride gas introduction tube and a phosgene introduction tube is charged with the inert solvent and the xylylene diamine, and the pressure of the inside of the reaction vessel is, for example, a normal pressure or more, and for example, 1 MPa (gauge pressure) or less, preferably 0.5 MPa (gauge pressure) or less, and the temperature thereof is, for example, 0°C or more, and for example, 150°C or less, preferably 120°C or less.

As the inert solvent, the above-described inert solvent is used, preferably, chlorinated aromatic hydrocarbons are used, more preferably, orthodichlorobenzene is used.

The mixing amount (total amount) of the inert solvent with respect to 100 parts by mass of the xylylene diamine is, for example, 400 parts by mass or more, preferably 500 parts by mass or more, and for example, 3000 parts by mass or less, preferably 2000 parts by mass or less.

Next, for example, 1 time mole or more, and for example, 5 times moles or less, preferably 3 times moles or less of the hydrogen chloride gas with respect to 1 mole of the amino group of the xylylene diamine is introduced. In this manner, a hydrochloride of the xylylene diamine is synthesized. The excessive hydrogen chloride used at this time is refined as needed, and can be reused in a preparation step of the hydrochloride.

The amine conversion rate of the xylylene diamine is, for example, 99.00 mol% or more, preferably 99.50 mol% or more, and for example, 99.99 mol% or less, preferably 99.90 mol% or less.

The amine conversion rate of the xylylene diamine can be measured in conformity with Examples to be described later.

The viscosity (at 120°C) of the hydrochloride of the xylylene diamine is, for example, 100 mPa·s or more, preferably 150 mPa·s or more, and for example, 500 mPa·s or less, preferably 300 mPa·s or less.

When the viscosity of the hydrochloride of the xylylene diamine is within the above-described range, the flowability thereof is excellent, and the liquid transfer properties at the time of the transfer of the hydrochloride of the xylylene diamine can be excellent.

The viscosity of the hydrochloride of the xylylene diamine can be measured in conformity with Examples to be described later.

The average particle size (number average value) of the hydrochloride of the xylylene diamine is, for example, 10 µm or more, preferably 20 µm or more, and for example, 100 µm or less, preferably 50 µm or less.

The average particle size (number average value) of the hydrochloride of the xylylene diamine can be measured in conformity with Examples to be described later.

Next, in this method, the reaction temperature is retained at, for example, 80°C or more, preferably 90°C or more, and for example, 180°C or less, preferably 160°C or less; the reaction pressure is retained at, for example, a normal pressure or more, preferably 0.05 MPa (gauge pressure) or more, and for example, 1.0 MPa (gauge pressure) or less, preferably 0.5 MPa (gauge pressure) or less; and the introduction of the phosgene is performed so that the total amount thereof is 1 time or more and 10 times or less of the stoichiometry over 1 hour or more and 10 hours or less. In this way, the hydrochloride of the xylylene diamine reacts with the phosgene.

In this manner, the xylylene diamine is subjected to isocyanate reaction, so that a xylylene diisocyanate composition containing the xylylene diisocyanate and the chloromethylbenzyl isocyanate that is a byproduct is obtained.

The progress of the reaction can be confirmed by the amount of the produced hydrogen chloride gas, and the state in which the slurry of the hydrochloride that is insoluble in the above-described inert solvent disappears and the reaction liquid is uniformly clear. The produced hydrogen chloride is, for example, emitted to the outside of the reaction vessel through the reflux condenser. At the time of the termination of the reaction, the excessive phosgene and the excessive hydrogen chloride that are dissolved by the above-described method are purged. Thereafter, the resulting product is cooled, and the inert solvent is distilled off under a reduced pressure. In this method, the inert reaction solvent, the hydrogen chloride, and the phosgene can be also retrieved, refined, and reused.

Furthermore, after the xylylene diamine is subjected to isocyanate reaction, and the xylylene diisocyanate is produced, the xylylene diamine can be retrieved from tar that is a reaction residue. An example of the retrieve method of the tar includes a method in which the tar reacts with supercritical water, supercritical carbon dioxide, water in a subcritical state, or carbon dioxide by a known method, so that the xylylene diamine is obtained.

After the termination of the reaction, the reaction liquid can be filtrated and desolvated as needed.

The hydrochloride conversion rate of the hydrochloride of the xylylene diamine is, for example, 99.00 mol% or more, preferably 99.50 mol% or more, and for example, 99.90 mol% or less, preferably 99.80 mol% or less.

The hydrochloride conversion rate of the hydrochloride of the xylylene diamine can be measured in conformity with Examples to be described later.

The generation rate of the chloromethylbenzyl isocyanate in the reaction liquid after the termination of the reaction is, for example, 0.01 mass% or more, preferably 0.05 mass% or more, more preferably 0.1 mass% or more, and for example, 1 mass% or less, preferably 0.5 mass% or less, more preferably 0.3 mass% or less.

The generation rate of the chloromethylbenzyl isocyanate in the reaction liquid after the termination of the reaction can be measured in conformity with Examples to be described later.

In this method, the xylylene diisocyanate composition is distilled and refined as needed, so that the content ratio of the chloromethylbenzyl isocyanate can be adjusted. The method for refinement is not particularly limited, and industrial isolation techniques such as distillation and crystallization can be used.

When the refinement is performed with the distillation, a distillation tower may be a plate tower or a filling tower. The distillation conditions can be appropriately set by the content ratio of the chloromethylbenzyl isocyanate that is necessary for the xylylene diisocyanate composition after the refinement. To be specific, the theoretical plate number of the distillation tower (filling tower) is, for example, 2 or more, preferably 5 or more, and for example, 60 or less, preferably 40 or less.

The tower top pressure of the distillation tower is, for example, 0.1 kPa or more, preferably 0.15 kPa or more, and for example, 4 kPa or less, preferably 2.5 kPa or less.

The tower top reflux ratio of the distillation tower is, for example, 0.01 or more, preferably 0.1 or more, and for example, 60 or less, preferably 40 or less.

The tower top temperature of the distillation tower is, for example, 110°C or more, preferably 120°C or more, and for example, 180°C or less, preferably 170°C or less. The tower bottom temperature (reboiler temperature) of the distillation tower is, for example, 120°C or more, preferably 130°C or more, and for example, 155°C or less, preferably 150°C or less.

Also, before the xylylene diisocyanate composition is distilled with a distillation tower, preliminary heating can be performed with a preheater. The preliminary heating temperature is, for example, 110°C or more, preferably 120°C or more, and for example, 155°C or less.

In this method, a fraction having the tower top distillation rate of the distillation tower within a range of, for example, 1 mass% or more, preferably 5 mass% or more, and for example, 99 mass% or less, preferably 95 mass% or less is retrieved.

In this manner, the content ratio of the chloromethylbenzyl isocyanate contained in the xylylene diisocyanate composition can be adjusted.

After the retrieve, the xylylene diisocyanate that is contained in a high boiling component containing a tower bottom residue at the tower bottom portion produced in the distillation step can be also retrieved by using a thin-film evaporator. Furthermore, a part of the high boiling component is brought back to the distillation step, and the xylylene diisocyanate is refined, so that the retrieve can be also performed.

Also, by adding the chloromethylbenzyl isocyanate to the xylylene diisocyanate composition, the content ratio of the chloromethylbenzyl isocyanate contained in the xylylene diisocyanate composition can be adjusted.

When the content ratio of the chloromethylbenzyl isocyanate in the xylylene diisocyanate composition is within the above-described specific range, the resin (described later) can be stably produced from the xylylene diisocyanate composition; improvement of the production efficiency of the resin (described later) can be achieved; and the resin (described later) having excellent yellowing resistance can be obtained.

Also, for example, a stabilizer can be added to the xylylene diisocyanate composition.

Examples of the stabilizer include an antioxidant, an acidic compound, a compound containing a sulfonamide group, and an organic phosphite.

As the stabilizer, preferably, an antioxidant, an acidic compound, and a compound containing a sulfonamide group are used.

The mixing ratio of the stabilizer is not particularly limited, and appropriately set in accordance with its necessity and use.

The xylylene diisocyanate composition of the present invention can also contain a derivative of the xylylene diisocyanate obtained by modifying the xylylene diisocyanate. To be more specific, examples thereof include multimer (dimer and trimer (for example, isocyanurate modified product)), biuret modified product, allophanate modified product, polyol modified product, oxadiazine trione modified product, carbodiimide modified product, and uretdione modified product of the xylylene diisocyanate.

The xylylene diisocyanate composition of the present invention contains the xylylene diisocyanate and the chloromethylbenzyl isocyanate, and the content ratio of the chloromethylbenzyl isocyanate is 10 mass ppm or more and 500 mass ppm or less.

Thus, the resin (described later) can be stably produced by using the xylylene diisocyanate composition of the present invention, improvement of the production efficiency of the resin (described later) can be achieved, and the obtained resin (described later) has excellent yellowing resistance.

Furthermore, the resin (described later) obtained from the xylylene diisocyanate composition of the present invention can retain its optical properties and improve the dyeability.

The present invention contains a polymerizable composition and the resin obtained by using the above-described xylylene diisocyanate composition.

To be specific, the polymerizable composition of the present invention contains the above-described xylylene diisocyanate composition and a polyol component and/or a polythiol component, and can be obtained by mixing those. The resin of the present invention can be obtained by allowing the polymerizable composition to react. That is, the resin of the present invention can be obtained by allowing the polyisocyanate component containing the above-described xylylene diisocyanate composition to react with the polyol component and/or the polythiol component. In view of production of an optical material, preferably, the above-described polyisocyanate component reacts with the polythiol component.

That is, the resin of the present invention is a reaction product (reaction product of the polymerizable composition) of the above-described xylylene diisocyanate composition (polyisocyanate component) with the polyol component and/or the polythiol component, and in view of production of an optical material, preferably, a reaction product of the above-described xylylene diisocyanate composition (polyisocyanate component) with the polythiol component.

The polyisocyanate component contains the above-described xylylene diisocyanate composition as an essential component.

The polyisocyanate component can contain another polyisocyanate as an optional component as long as it does not damage the excellent effect of the present invention. Examples of the other polyisocyanate include aliphatic polyisocyanate, alicyclic polyisocyanate, araliphatic polyisocyanate (excluding the xylylene diisocyanate), and aromatic polyisocyanate.

Examples of the aliphatic polyisocyanate include aliphatic diisocyanates such as trimethylene diisocyanate, tetramethylene diisocyanate, pentamethylene diisocyanate (PDI), hexamethylene diisocyanate (HDI), 1,2-propylene diisocyanate, 1,2-butylene diisocyanate, 2,3-butylene diisocyanate, 1,3-butylene diisocyanate, 2,4,4- or 2,2,4-trimethylhexamethylene diisocyanate, and 2,6-diisocyanatemethylcaproate.

Examples of the alicyclic polyisocyanate include alicyclic diisocyanates such as 1,3-cyclopentane diisocyanate, 1,4-cyclohexane diisocyanate, 1,3-cyclohexane diisocyanate, 3-isocyanatomethyl-3,5,5-trimethylcyclohexyl isocyanate (IPDI), 4,4'-methylenebis(cyclohexyl isocyanate), methyl-2,4-cyclohexane diisocyanate, methyl-2,6-cyclohexane diisocyanate, 1,3-bis(isocyanatomethyl)cyclohexane, 1,3-bis(isocyanatoethyl)cyclohexane, 1,4-bis(isocyanatomethyl)cyclohexane, 1,4-bis(isocyanatoethyl)cyclohexane, and 2,5- or 2,6-bis(isocyanatomethyl)norbornane (NBDI) and a mixture thereof.

Examples of the araliphatic polyisocyanate (excluding the xylylene diisocyanate) include araliphatic diisocyanates such as 1,3- or 1,4-tetramethylxylylene diisocyanate or a mixture thereof (TMXDI) and ω,ω'-diisocyanato-1,4-diethylbenzene.

Examples of the aromatic polyisocyanate include aromatic diisocyanates such as 2,4-tolylene diisocyanate, 2,6-tolylene diisocyanate, and an isomer mixture of the tolylene diisocyanate (TDI); 4,4'-diphenylmethane diisocyanate, 2,4'-diphenylmethane diisocyanate, 2,2'-diphenylmethane diisocyanate, and an optional isomer mixture of the diphenylmethane diisocyanate (MDI); toluylene diisocyanate; paraphenylene diisocyanate; and naphthalene diisocyanate.

Derivatives of the polyisocyanates can be also used in combination. To be more specific, examples thereof include multimer (dimer and trimer (for example, isocyanurate modified product)), biuret modified product, allophanate modified product, polyol modified product, oxadiazine trione modified product, carbodiimide modified product, and uretdione modified product of the polyisocyanate.

When the above-described xylylene diisocyanate composition and another polyisocyanate are used in combination, as the mixing ratio thereof, they are mixed at an optional mixing ratio in accordance with the use of the resin produced by using the polyisocyanate component.

In the present invention, examples of the polyol component include a low molecular weight polyol and/or a high molecular weight polyol.

The low molecular weight polyol is a compound having two or more hydroxyl groups and having a number average molecular weight of 60 or more and below 400. Examples thereof include dihydric alcohols such as ethylene glycol, propylene glycol, 1,3-propanediol, 1,4-butylene glycol, 1,3-butylene glycol, 1,2-butylene glycol, 1,5-pentanediol, 1,6-hexanediol, neopentyl glycol, alkane (7 to 22) diol, diethylene glycol, triethylene glycol, dipropylene glycol, 3-methyl-1,5-pentanediol, alkane-1,2-diol (C (carbon number, hereinafter the same) 17 to C20), isosorbide, 1,3- or 1,4-cyclohexanedimethanol and a mixture thereof, 1,4-cyclohexanediol, hydrogenated bisphenol A, 1,4-dihydroxy-2-butene, 2,6-dimethyl-1-octene-3,8-diol, and bisphenol A; trihydric alcohols such as glycerin and trimethylol propane; tetrahydric alcohols such as tetramethylolmethane (pentaerythritol) and diglycerin; pentahydric alcohols such as xylitol; hexahydric alcohols such as sorbitol, mannitol, allitol, iditol, dulcitol, altritol, inositol, and dipentaerythritol; heptahydric alcohols such as perseitol; and octahydric alcohols such as sucrose.

Also, an example of the low molecular weight polyol includes a polyalkylene oxide (including a random and/or block copolymer of two or more alkylene oxides) having a number average molecular weight of 60 or more and below 400 obtained by adding an alkylene oxide such as ethylene oxide and propylene oxide with these as an initiator.

The high molecular weight polyol is a compound having two or more hydroxyl groups and having a number average molecular weight of 400 or more and usually 20000 or less. Examples thereof include polyether polyol, polyester polyol, polycarbonate polyol, polyurethane polyol, epoxy polyol, vegetable oil polyol, polyolefin polyol, acrylic polyol, and vinyl monomer-modified polyol.

Examples of the polyether polyol include polyoxy (C2 to C3) alkylene polyol, polytetramethylene ether glycol, and polytrimethylene ether glycol.

An example of the polyoxy (C2 to C3) alkylene polyol includes an addition polymer (including a random and/or block copolymer of two or more alkylene oxides) of the C2 to C3 alkylene oxide such as ethylene oxide and propylene oxide with the above-described low molecular weight polyol as an initiator. An example of the polyoxy (C2 to C3) alkylene includes polyethylene glycol.

Examples of the polytetramethylene ether glycol include a ring-opening polymer obtained by cation polymerization of tetrahydrofuran and an amorphous polytetramethylene ether glycol obtained by copolymerizing the above-described dihydric alcohol with a polymerization unit of the tetrahydrofuran.

Also, an example thereof includes a polytetramethylene ether glycol derived from plants with tetrahydrofuran produced based on a plant material such as furfural as a starting material.

An example of the polytrimethylene ether glycol includes a polyol produced by condensation polymerization of 1,3-propanediol derived from plants.

An example of the polyester polyol includes a polycondensate obtained by allowing the above-described low molecular weight polyol (preferably, dihydric alcohol) to react with a polybasic acid under known conditions.

Examples of the polybasic acid include saturated aliphatic dicarboxylic acids such as oxalic acid, malonic acid, succinic acid, methylsuccinic acid, glutaric acid, adipic acid, 1,1-dimethyl-1,3-dicarboxypropane, 3-methyl-3-ethylglutaric acid, azelaic acid, sebacic acid, and other saturated aliphatic dicarboxylic acid (carbon number of 11 to 13); unsaturated aliphatic dicarboxylic acids such as maleic acid, fumaric acid, itaconic acid, and others; aromatic dicarboxylic acids such as orthophthalic acid, isophthalic acid, terephthalic acid, toluene dicarboxylic acid, naphthalene dicarboxylic acid, and others; alicyclic dicarboxylic acids such as hexahydrophthalic acid and others; other carboxylic acids such as dimer acid, hydrogenated dimer acid, HET acid, and others; anhydrides derived from the carboxylic acids such as oxalic anhydrides, succinic anhydrides, maleic anhydrides, phthalic anhydrides, 2-alkyl (C12 to C18) succinic anhydrides, tetrahydrophthalic anhydrides, and trimellitic anhydrides; and furthermore, acid halides derived from the carboxylic acids such as oxalyl dichlorides, adipic acid dichlorides, and sebacic acid dichlorides.

An example of the polyester polyol includes a vegetable oil-based polyester polyol obtained by subjecting the above-described low molecular weight polyol and the hydroxy carboxylic acid such as hydroxyl group-containing vegetable oil fatty acid (for example, castor oil fatty acid containing a ricinoleic acid or hydrogenated castor oil fatty acid containing a 12-hydroxystearic acid) to condensation reaction under known conditions.

Examples of the polyester polyol include a polycaprolactone polyol and a polyvalerolactone polyol obtained by subjecting lactones such as ε-caprolactone and γ-valerolactone to ring-opening polymerization with the above-described low molecular weight polyol (preferably, dihydric alcohol) as the initiator, and furthermore, a lactone polyester polyol obtained by copolymerizing these with the above-described dihydric alcohol.

Examples of the polycarbonate polyol include a ring-opening polymer of ethylene carbonate with the above-described low molecular weight polyol (preferably, dihydric alcohol) as an initiator and an amorphous polycarbonate polyol obtained by copolymerizing the dihydric alcohols such as 1,4-butanediol, 1,5-pentanediol, 3-methyl-1,5-pentanediol, and 1,6-hexanediol with the ring-opening polymer.

The polyurethane polyol can be obtained, for example, as polyester polyurethane polyol, polyether polyurethane polyol, polycarbonate polyurethane polyol, or polyester polyether polyurethane polyol by allowing the polyester polyol, the polyether polyol, and/or the polycarbonate polyol obtained by the description above to react with the above-described polyisocyanate (including the xylylene diisocyanate, hereinafter, the same) at an equivalent ratio (OH/NCO) of the hydroxyl group to the isocyanate group of above 1.

An example of the epoxy polyol includes an epoxy polyol obtained by allowing the above-described low molecular weight polyol to react with polyfunctional halohydrins such as epichlorohydrin and β-methylepichlorohydrin.

Examples of the vegetable oil polyol include hydroxyl group-containing vegetable oils such as castor oil and coconut oil. Also, examples thereof include a castor oil polyol and an ester-modified castor oil polyol obtained by allowing a castor oil polyol to react with a polypropylene polyol.

Examples of the polyolefin polyol include a polybutadiene polyol and a partially saponified ethylene-vinyl acetate copolymer.

An example of the acrylic polyol includes a copolymer obtained by copolymerizing a hydroxyl group-containing acrylate with a copolymerizable vinyl monomer that is copolymerizable with the hydroxyl group-containing acrylate.

Examples of the hydroxyl group-containing acrylate include 2-hydroxyethyl (meth)acrylate, hydroxypropyl (meth)acrylate, hydroxybutyl (meth)acrylate, 2,2-dihydroxymethylbutyl (meth)acrylate, polyhydroxyalkyl maleate, and polyhydroxyalkyl fumarate. Preferably, a 2-hydroxyethyl (meth)acrylate is used.

Examples of the copolymerizable vinyl monomer include alkyl (meth)acrylate monomers (carbon number of 1 to 12) such as methyl (meth)acrylate, ethyl (meth)acrylate, propyl (meth)acrylate, isopropyl (meth)acrylate, butyl (meth)acrylate, isobutyl (meth)acrylate, s-butyl (meth)acrylate, t-butyl (meth)acrylate, pentyl (meth)acrylate, isopentyl (meth)acrylate, hexyl (meth)acrylate, isononyl (meth)acrylate, 2-ethylhexyl (meth)acrylate, cyclohexyl acrylate, and isobornyl (meth)acrylate; aromatic vinyl monomers such as styrene, vinyltoluene, and α-methylstyrene; vinyl cyanides such as (meth)acrylonitrile; vinyl monomers containing a carboxyl group such as (meth)acrylic acid, fumaric acid, maleic acid, and itaconic acid or alkyl esters thereof; alkanepolyol poly(meth)acrylates such as ethyleneglycol di(meth)acrylate, butyleneglycol di(meth)acrylate, hexanediol di(meth)acrylate, oligoethyleneglycol di(meth)acrylate, trimethylolpropane di(meth)acrylate, and trimethylolpropane tri(meth)acrylate; and vinyl monomers containing an isocyanate group such as 3-(2-isocyanate-2-propyl)-α-methylstyrene.

The acrylic polyol can be obtained by copolymerizing the hydroxyl group-containing acrylate with the copolymerizable vinyl monomer under the presence of an appropriate solvent and a polymerization initiator.

Examples of the acrylic polyol include a silicone polyol and a fluorine polyol.

An example of the silicone polyol includes an acrylic polyol in which a silicone compound containing a vinyl group such as γ-methacryloxypropyltrimethoxysilane is blended as a copolymerizable vinyl monomer in the copolymerization of the above-described acrylic polyol.

An example of the fluorine polyol includes an acrylic polyol in which a fluorine compound containing a vinyl group such as tetrafluoroethylene and chlorotrifluoroethylene is blended as a copolymerizable vinyl monomer in the copolymerization of the above-described acrylic polyol.

The vinyl monomer-modified polyol can be obtained by allowing the above-described high molecular weight polyol to react with the vinyl monomer.

As the high molecular weight polyol, preferably, a high molecular weight polyol selected from polyether polyol, polyester polyol, and polycarbonate polyol is used.

Examples of the vinyl monomer include the above-described alkyl (meth)acrylate, vinyl cyanide, and vinylidene cyanide. These vinyl monomers can be used alone or in combination of two or more. Of these, preferably, an alkyl (meth)acrylate is used.

The vinyl monomer-modified polyol can be obtained by allowing the high molecular weight polyol to react with the vinyl monomer under the presence of a radical polymerization initiator (for example, persulfate, organic peroxide, or azo-based compound).

These polyol components can be used alone or in combination of two or more.

Examples of the polythiol component include aliphatic polythiol, aromatic polythiol, heterocycle-containing polythiol, aliphatic polythiol containing a sulfur atom in addition to a mercapto group, aromatic polythiol containing a sulfur atom in addition to a mercapto group, and heterocycle-containing polythiol containing a sulfur atom in addition to a mercapto group.

Examples of the aliphatic polythiol include methanedithiol, 1,2-ethanedithiol, 1,1-propanedithiol, 1,2-propanedithiol, 1,3-propanedithiol, 1,4-butanedithiol, 1,5-pentanedithiol, 2,2-propanedithiol, 1,6-hexanedithiol, 1,2,3-propanetrithiol, 1,1-cyclohexanedithiol, 1,2-cyclohexanedithiol, 2,2-dimethylpropane-1,3 -dithiol, 3,4-dimethoxybutane-1,2-dithiol, 2-methylcyclohexane-2,3-dithiol, 1-methylcyclohexane-2,3-dithiol, bicyclo[2,2,1]hepta-exo-cis-2,3-dithiol, tetrakis(mercaptomethyl)methane, 1,1-bis(mercaptomethyl)cyclohexane, thiomalate bis(2-mercaptoethylester), 2,3-dimercaptosuccinic acid(2-mercaptoethylester), 2,3-dimercapto-1-propanol(2-mercaptoacetate), 2,3-dimercapto-1-propanol(3-mercaptopropionate), diethyleneglycol bis(2-mercaptoacetate), diethyleneglycol bis(3-mercaptopropionate), 1,2-dimercaptopropylmethyl ether, 2,3-dimercaptopropylmethyl ether, 2,2-bis(mercaptomethyl)-1,3-propanedithiol, bis(2-mercaptoethyl)ether, ethyleneglycol bis(2-mercaptoacetate), ethyleneglycol bis(3-mercaptopropionate), trimethylolpropane bis(2-mercaptoacetate), trimethylolpropane bis(3-mercaptopropionate), 3-mercapto-1,2-propanediol bis(2-mercaptoacetate), 3-mercapto-1,2-propanediol di(3-mercaptopropionate), trimethylolpropane tris(2-mercaptoacetate), trimethylolpropane(3-mercaptopropionate), trimethylolethane tris(2-mercaptoacetate), trimethylolethane tris(3-mercaptopropionate), pentaerythritol tetrakis(2-mercaptoacetate), pentaerythritol terakis(3-mercaptopropionate), glycerin tris(2-mercaptoacetate), glycerin tris(3-mercaptopropionate), 1,4-cyclohexanediol bis(2-mercaptoacetate), and 1,4-cyclohexanediol bis(3-mercaptopropionate).

Examples of the aromatic polythiol include 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(mercaptoethyl)benzene, 1,3-bis(mercaptoethyl)benzene, 1,4-bis(mercaptoethyl)benzene, 1,2-bis(mercaptomethyleneoxy)benzene, 1,3-bis(mercaptomethyleneoxy)benzene, 1,4-bis(mercaptomethyleneoxy)benzene, 1,2-bis(mercaptoethyleneoxy)benzene, 1,3-bis(mercaptoethyleneoxy)benzene, 1,4-bis(mercaptoethyleneoxy)benzene, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl)benzene, 1,3,5 -tris(mercaptomethyl)benzene, 1,2,3-tris(mercaptoethyl)benzene, 1,2,4-tris(mercaptoethyl)benzene, 1,3,5-tris(mercaptoethyl)benzene, 1,2,3-tris(mercaptomethyleneoxy)benzene, 1,2,4-tris(mercaptomethyleneoxy)benzene, 1,3,5-tris(mercaptomethyleneoxy)benzene, 1,2,3-tris(mercaptoethyleneoxy)benzene, 1,2,4-tris(mercaptoethyleneoxy)benzene, 1,3,5-tris(mercaptoethyleneoxy)benzene, 1,2,3,4-tetramercaptobenzene, 1,2,3,5-tetramercaptobenzene, 1,2,4,5-tetramercaptobenzene, 1,2,3,4-tetrakis(mercaptomethyl)benzene, 1,2,3,5-tetrakis(mercaptomethyl)benzene, 1,2,4,5-tetrakis(mercaptomethyl)benzene, 1,2,3,4-tetrakis(mercaptoethyl)benzene, 1,2,3,5-tetrakis(mercaptoethyl)benzene, 1,2,4,5-tetrakis(mercaptoethyl)benzene, 1,2,3,4-tetrakis(mercaptomethyleneoxy)benzene, 1,2,3,5-tetrakis(mercaptomethyleneoxy)benzene, 1,2,4,5-tetrakis(mercaptoethyleneoxy)benzene, 1,2,3,4-tetrakis(mercaptoethyleneoxy)benzene, 1,2,3,5-tetrakis(mercaptoethyleneoxy)benzene, 1,2,4,5-tetrakis(mercaptoethyleneoxy)benzene, 2,2'-dimercaptobiphenyl, 4,4'-dimercaptobiphenyl, 4,4'-dimercaptobibenzyl, 2,5-toluenedithiol, 3,4-toluenedithiol, 1,4-naphthalenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-di(p-methoxyphenyl)propane-2,2-dithiol, 1,3-diphenylpropane-2,2-dithiol, phenylmethane-1,1-dithiol, and 2,4-di(p-mercaptophenyl)pentane.

Examples of the heterocycle-containing polythiol include 2-methylamino-4,6-dithiol-sym-triazine, 2-ethylamino-4,6-dithiol-sym-triazine, 2-amino-4,6-dithiol-sym-triazine, 2-morpholino-4,6-dithiol-sym-triazine, 2-cyclohexylamino-4,6-dithiol-sym-triazine, 2-methoxy-4,6-dithiol-sym-triazine, 2-phenoxy-4,6-dithiol-sym-triazine, 2-thiobenzeneoxy-4,6-dithiol-sym-triazine, and 2-thiobutyloxy-4,6-dithiol-sym-triazine.

Examples of the aliphatic polythiol containing a sulfur atom in addition to a mercapto group include bis(mercaptomethyl)sulfide, bis(mercaptoethyl)sulfide, bis(mercaptopropyl)sulfide, bis(mercaptomethylthio)methane, bis(2-mercaptoethylthio)methane, bis(3 -mercaptopropylthio)methane, 1,2-bis(mercaptomethylthio)ethane, 1,2-bis(2-mercaptoethylthio)ethane, 1,2-bis(3-mercaptopropyl)ethane, 1,3-bis(mercaptomethylthio)propane, 1,3-bis(2-mercaptoethylthio)propane, 1,3-bis(3-mercaptopropylthio)propane, 1,2,3-tris(mercaptomethylthio)propane, 1,2,3-tris(2-mercaptoethylthio)propane, 1,2,3-tris(3-mercaptopropylthio)propane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl)sulfide, 2,5-dimercapto-1,4-dithiane, 2,5-dimercaptomethyl-1,4-dithiane, bis(mercaptomethyl)disulfide, bis(mercaptoethyl)disulfide, and bis(mercaptopropyl)disulfide and thioglycolic acid thereof and ester of mercaptopropionate, hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercaptopropionate), hydroxypropyl sulfide bis(2-mercaptoacetate), hydroxypropyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), hydroxypropyl disulfide bis(2-mercaptoacetate), hydroxypropyl disulfide bis(3-mercaptopropionate), 2-mercaptoethyl ether bis(2-mercaptoacetate), 2-mercaptoethyl ether bis(3-mercaptopropionate), 1,4-dithiane-2,5-diol bis(2-mercaptoacetate), 1,4-dithiane-2,5-diol bis(3-mercaptopropionate), thioglycolic acid bis(2-mercaptoethylester), thiodipropionic acid bis(2-mercaptoethylester), 4,4-thiodibutylic acid bis(2-mercaptoethylester), dithioglycolic acid bis(2-mercaptoethylester), dithiodipropionic acid bis(2-mercaptoethylester), 4,4-dithiodibutylic acid bis(2-mercaptoethylester), thioglycolic acid bis(2,3-dimercaptopropylester), thiodipropionic acid bis(2,3-dimercaptopropylester), dithioglycolic acid bis(2,3-dimercaptopropylester), dithiodipropionic acid bis(2,3-dimercaptopropylester), 1,2-bis[(2-mercaptoethyl)thio]-3-mercaptopropane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 1,1,3,3-tetrakis(mercaptomethylthio)propane, 4,6-bis(mercaptomethylthio)-1,3-dithiane, and 2-(2,2-bis(mercaptomethylthio)ethyl)-1,3-dithiethane.

Examples of the aromatic polythiol containing a sulfur atom in addition to a mercapto group include 1,2-bis(mercaptomethylthio)benzene, 1,3-bis(mercaptomethylthio)benzene, 1,4-bis(mercaptomethylthio)benzene, 1,2-bis(mercaptoethylthio)benzene, 1,3-bis(mercaptoethylthio)benzene, 1,4-bis(mercaptoethylthio)benzene, 1,2,3-tris(mercaptomethylthio)benzene, 1,2,4-tris(mercaptomethylthio)benzene, 1,3,5-tris(mercaptomethylthio)benzene, 1,2,3-tris(mercaptoethylthio)benzene, 1,2,4-tris(mercaptoethylthio)benzene, 1,3,5-tris(mercaptoethylthio)benzene, 1,2,3,4-tetrakis(mercaptomethylthio)benzene, 1,2,3,5-tetrakis(mercaptomethylthio)benzene, 1,2,4,5-tetrakis(mercaptomethylthio)benzene, 1,2,3,4-tetrakis(mercaptoethylthio)benzene, 1,2,3,5-tetrakis(mercaptoethylthio)benzene, and 1,2,4,5-tetrakis(mercaptoethylthio)benzene and a nucleus alkylation thereof.

Examples of the heterocycle-containing polythiol containing a sulfur atom in addition to a mercapto group include 3,4-thiophenedithiol and 2,5-dimercapto-1,3,4-thiadiazole and thioglycolic acid thereof and ester of mercaptopropionate.

As the polythiol component, furthermore, for example, halogen substituted products such as chlorine substituted product and bromine substituted product of the polythiol are used.

These polythiol components can be used alone or in combination of two or more.

As the polythiol component, preferably, an aliphatic polythiol containing a sulfur atom in addition to a mercapto group is used, more preferably, 1,2-bis[(2-mercaptoethyl)thio]-3-mercaptopropane, 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane and a mixture thereof are used, further more preferably, a mixture of 1,2-bis[(2-mercaptoethyl)thio]-3-mercaptopropane, 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane is used, particularly preferably, a mixture of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,-dimercapto-3,6,9-trithiaundecane is used.

The resin of the present invention is produced by the reaction of the above-described polyisocyanate component with at least any one of the above-described polyol component and the above-described polythiol component.

In the production of the resin of the present invention, the polyisocyanate component contains the above-described xylylene diisocyanate composition, and in view of production of an optical material, preferably, the above-described xylylene diisocyanate composition is used alone.

The hydroxyl value of the polyol component and/or the polythiol component in the production of the resin of the present invention is, for example, 10 mgKOH/g or more, preferably 100 mgKOH/g or more, and for example, 1240 mgKOH/g or less, preferably 940 mgKOH/g or less, more preferably 500 mgKOH/g or less; the number average molecular weight thereof is, for example, 90 or more, preferably 400 or more, and for example, 5000 or less, preferably 3000 or less; and the average functionality thereof is, for example, 2 or more, preferably above 2, and usually 8 or less, preferably 4 or less.

The hydroxyl value can be obtained by an acetylation method and a phthalation method in conformity with A method or B method of JIS K 1557-1 (in 2007), and the relationship between the hydroxyl value and the hydroxyl equivalent is shown by the following formula (1) (hereinafter, the same).$Hydroxyl value = 56100 / hydroxyl equivalent$ (1)

The number average molecular weight can be obtained from the hydroxyl equivalent and the average functionality, and the average functionality can be obtained from the following formula (2) (hereinafter, the same).$Average functionality = total sum of \left(functionality of each of the polyols and/or each of the polythiols\times number of equivalent\right) / total sum of the number of equivalent of each of the polyols and / or each of the polythiols$ (2)

The hydroxyl value of the polyol component and/or the polythiol component, among all, in the production of the resin of the present invention as an optical material is, for example, 280 mgKOH/g or more, preferably 400 mgKOH/g or more, and for example, 1240 mgKOH/g or less, preferably 940 mgKOH/g or less; the number average molecular weight thereof is, for example, 90 or more, preferably 100 or more, and for example, 1000 or less, preferably 800 or less; and the average functionality thereof is, for example, above 2, preferably 2.5 or more, and usually 5.0 or less, preferably below 4.0.

When the hydroxyl value, the number average molecular weight, and the average functionality of the resin that is produced as an optical material are within the above-described range, the shock resistance and the heat resistance of the resin can be improved.

Furthermore, for example, a known polyamine, a known monool, and a known monoamine can be blended in the polyol component and/or the polythiol component at an appropriate ratio as needed.

The resin of the present invention can be, for example, produced by a polymerization method such as bulk polymerization and solution polymerization, and when the resin of the present invention is produced as an optical material, preferably, a bulk polymerization is used.

In the bulk polymerization, for example, under a nitrogen gas stream, the polyol component and/or the polythiol component are/is added to the polyisocyanate component, while the polyisocyanate component is stirred, so that a polymerizable composition is prepared. Then, a xylylene diisocyanate composition reacts with the polyol component and/or the polythiol component at a reaction temperature of, for example, 50°C or more, and for example, 250°C or less, preferably 200°C or less for a reaction time of, for example, 0.5 hours or more, and for example, 20 hours or less, preferably 15 hours or less.

In the solution polymerization, a polyisocyanate component and the polyol component and/or the polythiol component are added to an organic solvent, so that a polymerizable composition is prepared. Then, a xylylene diisocyanate composition reacts with the polyol component and/or the polythiol component at a reaction temperature of, for example, 50°C or more, and for example, 120°C or less, preferably 100°C or less for a reaction time of, for example, 0.5 hours or more, and for example, 20 hours or less, preferably 15 hours or less.

Examples of the organic solvent include ketones such as acetone, methyl ethyl ketone, methyl isobutyl ketone, and cyclohexanone; nitriles such as acetonitrile; alkyl esters such as methyl acetate, ethyl acetate, butyl acetate, and isobutyl acetate; aliphatic hydrocarbons such as n-hexane, n-heptane, and octane; alicyclic hydrocarbons such as cyclohexane and methyl cyclohexane; aromatic hydrocarbons such as toluene, xylene, and ethyl benzene; glycol ether esters such as methyl cellosolve acetate, ethyl cellosolve acetate, methyl carbitol acetate, ethyl carbitol acetate, ethylene glycol ethyl ether acetate, propylene glycol methyl ether acetate, 3-methyl-3-methoxybutylacetate, and ethyl-3-ethoxypropionate; ethers such as diethyl ether, tetrahydrofuran, and dioxane; halogenated aliphatic hydrocarbons such as methyl chloride, methylene chloride, chloroform, carbon tetrachloride, methyl bromide, methylene iodide, and dichloroethane; and aprotic polar solvents such as N-methyl pyrrolidone, dimethyl formamide, N,N'-dimethylacetamide, dimethyl sulfoxide, and hexamethylphosphonylamide.

Furthermore, in the above-described polymerization reaction, for example, a known urethane-formation catalyst such as amines and organic metal compound may be added as needed.

Examples of the amines include tertiary amines such as triethylamine, triethylenediamine, bis-(2-dimethylaminoethyl) ether, and N-methylmorpholine; quaternary ammonium salts such as tetraethylhydroxylammonium; and imidazoles such as imidazole and 2-ethyl-4-methylimidazole.

Examples of the organic metal compound include organic tin compounds such as tin acetate, tin octylate, tin oleate, tin laurate, dibutyltin diacetate, dimethyltin dilaurate, dibutyltin dilaurate, dibutyltin dimercaptide, dibutyltin maleate, dibutyltin dilaurate, dibutyltin dineodecanoate, dioctyltin dimercaptide, dioctyltin dilaurate, and dibutyltin dichloride; organic lead compounds such as lead octanoate and lead naphthenate; organic nickel compounds such as nickel naphthenate; organic cobalt compounds such as cobalt naphthenate; organic copper compounds such as copper octenoate; organic bismuth compounds such as bismuth octylate and bismuth neodecanoate; organic zirconium compounds such as zirconium acetylacetone chelate; organic titanium compounds such as titanium acetoacetic acid chelate and bis(2-ethylhexanoic acid) titanium; and organic iron compounds such as iron acetylacetone chelate.

Furthermore, examples of the urethane-formation catalyst include potassium salts such as potassium carbonate, potassium acetate, and potassium octylate.

These urethane-formation catalysts can be used alone or in combination of two or more.

As the urethane-formation catalyst, preferably, an organic metal compound is used, more preferably, an organic tin compound is used, further more preferably, dibutyltin dichloride is used.

The mixing ratio of the urethane-formation catalyst with respect to 100 parts by mass of the total amount of the polyisocyanate component and the polyol component and/or the polythiol component is, for example, 0.0001 parts by mass or more, preferably 0.001 parts by mass or more, and for example, 0.5 parts by mass or less, preferably 0.1 parts by mass or less.

In the bulk polymerization and the solution polymerization, for example, the polyisocyanate component and the polyol component and/or the polythiol component are blended so that the equivalent ratio of the isocyanate group in the polyisocyanate component with respect to the hydroxyl group and the mercapto group in the polyol component and/or the polythiol component (isocyanate group (NCO)/hydroxyl group (OH) and mercapto group (SH)) is, for example, 0.75 or more, preferably 0.9 or more, and for example, 1.5 or less, preferably 1.3 or less.

When the above-described polymerization reaction is performed more industrially, for example, the resin can be obtained by a known method such as one shot method or pre-polymer method, and when the resin of the present invention is produced as an optical material, preferably, the resin is produced by a one shot method.

In the one shot method, for example, the polyisocyanate component and the polyol component and/or the polythiol component are formulated (mixed) so that the equivalent ratio of the isocyanate group in the polyisocyanate component with respect to the hydroxyl group and the mercapto group in the polyol component and/or the polythiol component (isocyanate group (NCO)/hydroxyl group (OH) and mercapto group (SH)) is, for example, 0.75 or more, preferably 0.9 or more, and for example, 1.5 or less, preferably 1.3 or less to be then subjected to curing reaction at, for example, a room temperature or more, and for example, 250°C or less, preferably 200°C or less, for, for example, 5 minutes or more, preferably 4 hours or more, and for example, 72 hours or less, preferably 24 hours or less. The curing temperature may be fixed, or can be gradually increased or cooled.

In the curing reaction, the polyisocyanate component and the polyol component and/or the polythiol component are mixed; thereafter, the air therein is released and/or filtrated as needed; and then, the obtained mixture is injected into a preliminary mold.

After the mixture is injected into the mold to react at, for example, 50°C or more, and for example, 150°C or less for, for example, 5 hours or more, and for example, 24 hours or less, and then, is removed from the mold, the resin that is molded into a desired shape can be obtained. After the demolding, the resulting product can be annealed at, for example, 100°C or more, and for example, 150°C or less for, for example, 2 hours or more, and for example, 8 hours or less as needed, and can be also aged at, for example, a room temperature within about 7 days as needed.

In the prepolymer method, for example, first, the polyisocyanate component reacts with a part of the polyol component and/or the polythiol component, thereby synthesizing an isocyanate group-terminated prepolymer having an isocyanate group at the end of the molecule. Next, the obtained isocyanate group-terminated prepolymer reacts with a remaining portion of the polyol component and/or the polythiol component to be subjected to curing reaction. In the prepolymer method, the remaining portion of the polyol component and/or the polythiol component is used as a chain extension agent.

To synthesize the isocyanate group-terminated prepolymer, the polyisocyanate component and a part of the polyol component and/or the polythiol component are formulated (mixed) so that the equivalent ratio of the isocyanate group in the polyisocyanate component with respect to the hydroxyl group and the mercapto group in a part of the polyol component and/or the polythiol component (isocyanate group (NCO)/hydroxyl group (OH) and mercapto group (SH)) is, for example, above 1.0, preferably 1.1 or more, more preferably 1.3 or more, and for example, 20 or less, preferably 10 or less, more preferably 6 or less to then react in a reaction vessel at, for example, a room temperature or more, preferably 50°C or more, and for example, 150°C or less, preferably 120°C or less for, for example, 0.5 hours or more, preferably 2 hours or more, and for example, 18 hours or less, preferably 10 hours or less. In the reaction, the above-described urethane-formation catalyst may be added as needed, or after the termination of the reaction, the unreacted polyisocyanate component can be also removed by, for example, a known removing method such as distillation and extraction as needed.

Next, to react the obtained isocyanate group-terminated prepolymer with the remaining portion of the polyol component and/or the polythiol component, the isocyanate group-terminated prepolymer and the remaining portion of the polyol component and/or the polythiol component are formulated (mixed) so that the equivalent ratio of the isocyanate group in the isocyanate group-terminated prepolymer with respect to the hydroxyl group and the mercapto group in the remaining portion of the polyol component and/or the polythiol component (isocyanate group (NCO)/hydroxyl group (OH) and mercapto group (SH)) is, for example, 0.75 or more, preferably 0.8 or more, and for example, 1.3 or less, preferably 1.2 or less to be then subjected to curing reaction at, for example, a room temperature or more, and for example, 250°C or less, preferably 200°C or less for, for example, 5 minutes or more, preferably 1 hour or more, and for example, 72 hours or less, preferably 24 hours or less.

In the curing reaction, the isocyanate group-terminated prepolymer and/or the remaining portion of the polyol component and/or the polythiol component are mixed; thereafter, the air therein is released and/or filtrated as needed; and then, the obtained mixture is injected into a mold.

After the mixture is injected into the mold to react at, for example, 50°C or more, and for example, 150°C or less for, for example, 5 hours or more, and for example, 24 hours or less, and then, is removed from the mold, the resin that is molded into a desired shape can be obtained. After the demolding, the resulting product can be annealed at, for example, 100°C or more, and for example, 150°C or less for, for example, 2 hours or more, and for example, 8 hours or less as needed, and can be also aged at, for example, a room temperature within about 7 days as needed.

In the production of the resin of the present invention, a known additive can be also further blended at an appropriate ratio as needed. Examples thereof include internal mold release agents, bluing agents, plasticizers, anti-blowing agents, leveling agents, matting agents, flame retardants, thixotropic agents, tackifiers, thickeners, lubricants, antistatics, sufcactants, reaction retarders, dehydrating agents, antioxidants, ultraviolet absorbers, hydrolysis inhibitors, and weathering stabilizers. These additives may be added at the time of the synthesis of each of the components, at the time of the mixture and the dissolution of each of the components, or furthermore, after the synthesis.

Among all, in the production of the resin of the present invention as an optical material, preferably, an internal mold release agent and an ultraviolet absorber are added.

Examples of the internal mold release agent include phosphate release agents, alkyl phosphate release agents, and fatty acid ester release agents. Preferably, a phosphate release agent is used. By blending the internal mold release agent, the resin that is easily capable of being released from a mold can be obtained.

An example of the phosphate release agent includes ZELEC UN (manufactured by Stepan Company).

These internal mold release agents can be used alone or in combination of two or more.

The mixing ratio of the internal mold release agent with respect to 100 parts by mass of the total amount of the polyisocyanate component and the polyol component and/or the polythiol component is, for example, 0.01 parts by mass or more, preferably 0.05 parts by mass or more, and for example, 10 parts by mass or less, preferably 5 parts by mass or less.

Examples of the ultraviolet absorber include benzotriazole compounds (to be specific, Tinuvin 571, Tinuvin 213, Tinuvin 234, and Tinuvin P (hereinabove, manufactured by BASF SE)), formamidine compounds (to be specific, Zikasorb R, Zikasorb BS, ZIKA-FA02, ZIKA-FUA, ZIKA-FUV, ZIKA-UVS3, and ZIKA-UVS4 (hereinabove, manufactured by ZIKO)), and Biosorb 583 (manufactured by SAKAI CHEMICAL INDUSTRY CO., LTD.).

These ultraviolet absorbers can be used alone or in combination of two or more.

The mixing ratio of the ultraviolet absorber with respect to 100 parts by mass of the total amount of the polyisocyanate component and the polyol component and/or the polythiol component is, for example, 0.01 parts by mass or more, preferably 0.06 parts by mass or more, and for example, 0.10 parts by mass or less, preferably 0.08 parts by mass or less.

When the obtained resin is used for a polarized lens, in the above-described molding method, for example, insert molding is used, that is, a polarized film is set in a mold in advance, and mixed materials (the polyisocyanate component and the polyol component and/or the polythiol component) can be also injected into the mold.

The resin of the present invention thus obtained has excellent yellowing resistance and excellent production efficiency.

Furthermore, the resin of the present invention retains its optical properties and can improve the dyeability.

The yellow index value (YI. value) (measured in conformity with Examples to be described later) of the resin of the present invention is, for example, 4.5 or more, and usually 5.0 or less, preferably 4.7 or less.

The specific gravity (measured in conformity with Examples to be described later) at 20°C of the resin of the present invention is, for example, 1.1 or more, preferably 1.2 or more, and for example, 2.0 or less, preferably 1.5 or less.

The refractive index (measured in conformity with Examples to be described later) at 20°C of the resin of the present invention is, for example, 1.50 or more, preferably 1.60 or more, and usually 2.0 or less.

The Abbe number (measured in conformity with Examples to be described later) at 20°C of the resin of the present invention is, for example, 30 or more, preferably 31 or more, and for example, 40 or less, preferably 35 or less.

The light transmittance (638 nm) (measured in conformity with Examples to be described later) of the resin of the present invention after dying (ref: Examples to be described later) is, for example, 60.0% or more, and for example, 64.0% or less, preferably 62.0% or less, more preferably 61.5% or less.

The light transmittance (567 nm) (measured in conformity with Examples to be described later) of the resin of the present invention after dying (ref: Examples to be described later) is, for example, 55.0% or more, and for example, 60.0% or less, preferably 59.0% or less, more preferably 58.0% or less.

The light transmittance (452 nm) (measured in conformity with Examples to be described later) of the resin of the present invention after dying (ref: Examples to be described later) is, for example, 55.0% or more, and for example, 60.0% or less, preferably 58.0% or less, more preferably 56.5% or less.

The heat resistance (measured in conformity with Examples to be described later) of the resin of the present invention is, for example, 104.0°C or more, and for example, 105.0°C or less, preferably 104.2°C or less.

The resin of the present invention can be used for uses of coating materials (paints, adhesives), elastomers, foams, and optical materials, among all, the resin of the present invention has excellent yellowing resistance, so that it is preferably used for optical materials.

Thus, when the resin of the present invention is produced as an optical material, for example, it can be preferably used for optical lenses such as transparent lens, sunglass lens, polarized lens, spectacle lens, camera lens, pickup lens, and contact lens; optical components such as lighting panel for car, head light lens, lamp cover for head light and tail light, optical element, optical disk, organic EL, and LED; and optical products such as decorative illumination for signboard, optical fiber, glass substitute, interlayer of laminated glass, or windshield of aircraft, wall of large water tank, transparent roof material, glazing material, transparent member of daily necessities, protective glasses, food, defensive shield, vehicle safety related parts, lighting component, smartphone, and tablet.

### Examples

The specific numerical values in mixing ratio (content ratio), property value, and parameter used in the following description can be replaced with upper limit values (numerical values defined as "or less" or "below") or lower limit values (numerical values defined as "or more" or "above") of corresponding numerical values in mixing ratio (content ratio), property value, and parameter described in the above-described "DESCRIPTION OF EMBODIMENTS".

In the following Preparation Examples, Examples, and Comparative Examples, the measurement was performed by the following method.

### 1. Measurement Method

### (Amine Conversion Rate of Xylylene Diamine)

An unreacted xylylene diamine (hereinafter, referred to as a remaining amine) that remains in a reaction liquid (slurry) after the termination of the synthesis reaction of the hydrochloride of the xylylene diamine was subjected to neutralization titration, thereby obtaining the number of moles of the remaining amine. The amine conversion rate of the xylylene diamine was calculated from the obtained result and the number of moles of the charged xylylene diamine (hereinafter, referred to as a charged amine) by the following formula (3).$Amine conversin rate = \left(\left(number of moles of charged amine-number of moles of remaining amine\right)/number of moles of charged amine\right) \times 100$ (3)

### (Generation Rate and Content Ratio of Chloromethylbenzyl Isocyanate in Xylylene Diisocyanate Composition)

First, a chloromethylbenzyl isocyanate having a purity of 99 mol% was used as a reference material, and a calibration curve was obtained from the area value of the gas chromatograph obtained from the following gas chromatograph analytical conditions.

Next, the reaction liquid after the termination of the reaction of the hydrochloride of the xylylene diamine with the phosgene was analyzed with a gas chromatography under the following conditions, thereby obtaining the number of moles of the chloromethylbenzyl isocyanate. The obtained result was converted into the mass, and divided by the mass of the reaction liquid after the termination of the reaction of the hydrochloride of the xylylene diamine and the phosgene, thereby obtaining the generation rate of the chloromethylbenzyl isocyanate in the xylylene diisocyanate composition.
Device: GC-2014 (manufactured by Shimadzu Corporation)
Column: DB-1, 0.53 mm of inner diameter × 60 mm of length × 1.5 µm of film thickness
(manufactured by Agilent Technologies Japan, Ltd.)
Oven temperature: temperature rising at 3°C/min from 130°C to 220°C, after reaching 220°C, temperature rising at 10°C/min until 300°C
Split ratio: no split (total amount injected)
Inlet temperature: 280°C
Detector temperature: 300°C
Carrier gas: N₂
Carrier gas flow rate: 8 ml/min
Detection method: FID
(Purity of Xylylene Diisocyanate)

The xylylene diisocyanate composition that was eventually obtained was analyzed with the gas chromatography in the same manner as described above, thereby obtaining the number of moles of the xylylene diisocyanate. The obtained result was converted into the mass, and divided by the mass of the xylylene diisocyanate composition, thereby obtaining the content ratio (purity) of the xylylene diisocyanate in the xylylene diisocyanate composition.

### (Hydrochloride Conversion Rate of Hydrochloride of Xylylene Diamine)

A residue of reaction filtered liquid obtained by filtration after the reaction of the hydrochloride of the xylylene diamine with the phosgene was subjected to neutralization titration, thereby obtaining the number of moles of the remaining hydrochloride of the xylylene diamine (hereinafter, referred to as a remaining hydrochloride). The conversion rate was calculated from the obtained result and the number of moles of the charged hydrochloride (hereinafter, referred to as a charged hydrochloride) by the following formula (4).$Hydrochloride conversion rate = \left(\left(number of moles of charged hydrochloride-number of moles of remaining hydrochloride\right)/number of moles of charged hydrochloride\right) \times 100$ (4)

### (Measurement of Viscosity of Hydrochloride of Xylylene Diamine)

A reaction liquid (slurry) after the termination of the synthesis reaction of the hydrochloride of the xylylene diamine was measured in a vessel, and the measurement temperature thereof was increased to 120°C. When the temperature thereof reached 120°C, the viscosity thereof was measured with a No.2 rotor of an LVT viscometer manufactured by BROOKFIELD, and the indicated value was multiplied by a coefficient, so that the viscosity was calculated.

### (Measurement of Average Particle Size (Number Average Value) of Hydrochloride of Xylylene Diamine)

A small amount of the reaction liquid (slurry) after the termination of the synthesis reaction of the hydrochloride of the xylylene diamine was extracted, and measured in an acetonitrile solvent with a laser diffraction particle size distribution measuring device: SALD-2100 manufactured by Shimadzu Corporation. The measured particle size was the number average value of the whole particle size.

### (Content Ratio of Chloromethylbenzyl Isocyanate in Xylylene Diisocyanate Composition)

After dissolving the xylylene diisocyanate composition in chloroform, the solution was analyzed with the gas chromatography in the same manner as described above, thereby obtaining the number of moles of the chloromethylbenzyl isocyanate. The obtained result was converted into the mass, and divided by the mass of the xylylene diisocyanate composition, thereby obtaining the content ratio of the chloromethylbenzyl isocyanate in the xylylene diisocyanate composition.

### (Calculation of Yellow Index Value (Y.I. Value) of Resin)

Each of the resins was produced as a circular flat plate plastic lens having a thickness of 9 mm and a diameter of 75 mm, and the chromaticity coordinate x, y was measured by using a color difference meter: CT-210 manufactured by Minolta Co., Ltd.. The Y.I. value was calculated based on the values of x and y that were the measurement result by the following formula (5).

There is a correlation such that the less the Y.I. value was, the better the color phase of the plastic lens was, and the larger the Y.I. value was, the worse the color phase was.$Y . I . value = \left(234\times x+106\times y+106\right) / y$ (5)

### (Measurement of Specific Gravity of Resin)

The specific gravity of the resin was measured by an Archimedes method.

### (Measurement of Optical Properties of Resin)

Each of the refractive index (nₑ) and the Abbe number (vₑ) at 20°C with a wavelength of 546.1 nm (mercury e-line) was measured by using a refractometer: KPR-20 (manufactured by Kalnew Optical Industrial Co., Ltd.).

### (Evaluation Method of Dyeability of Resin)

In 3000 g of pure water, 2.3 g of "FSP Red E-A" (manufactured by Futaba Industrial Co., Ltd., dye), 1.5 g of "FSP Yellow P-E" (manufactured by Futaba Industrial Co., Ltd., dye), 6.0 g of "FSP Blue AUL-S" (manufactured by Futaba Industrial Co., Ltd., dye), 6.0 g of "NICCA SUNSOLT #7000" (manufactured by NICCA CHEMICAL CO., LTD., dyeing dispersant), and 6.0 g of "DK-CN" (manufactured by DAIWA CHEMICAL INDUSTRIES CO., LTD., dyeing auxiliary) were added, thereby preparing a dying dispersion liquid. The resin having a thickness of 9 mm was immersed therein at 80°C for 30 minutes to be dyed. The light transmittance (%) at 638 nm, 567 nm, and 452 nm of the dyed resin was measured.

As the resin was more dyed, the light was absorbed by the dye, so that the lower the light transmittance at each of the wavelengths was, the more excellent the dyeability was.

### (Evaluation Method of Heat Resistance of Resin)

The glass transition temperature (Tg) was measured by a TMA penetration method (load: 50 g, diameter of pin top: 0.5 mm, temperature rising rate: 10°C/min) by using a thermomechanical analysis device: TMA-60 (manufactured by Shimadzu Corporation). The glass transition temperature was defined as an indicator of the heat resistance.

### 2. Preparation of Xylylene Diisocyanate Composition

### (Preparation Example 1)

An autoclave (reaction vessel) including a pressure controller that was equipped with a reflux cooling tube, a stirring blade, a thermometer, a hydrogen chloride gas introduction tube, a phosgene introduction tube, a material tank, and a material charging pump was used. In the reaction vessel, the value of the diameter (D1) of the stirring blade/the inner diameter (D2) of the reaction vessel was 0.7; the value of the tank diameter (D)/the tank length (L) was 0.59; and the inner volume of the reaction vessel was 2 m³. The reaction vessel was charged with 846 kg of orthodichlorobenzene as an inert solvent, and the material tank was charged with 136.2 kg (1.0 kmol) of m-xylylenediamine and 621 kg of orthodichlorobenzene (the concentration of the entire amine: 8.5 weight%).

Next, after the temperature of the inside of the reaction vessel was increased to 120°C, the inner pressure was adjusted to be higher than the atmospheric pressure by 0.01 MPa. Then, the reaction vessel started to be charged with a hydrogen chloride gas at a rate of 43.8 kg/hr through the hydrogen chloride gas introduction tube, and simultaneously, started to be charged with the m-xylylenediamine that was diluted with the inert solvent from the material tank at a rate of 379 kg/hr through the material charging pump, so that the total amount was charged over 2 hours. Thereafter, furthermore, the hydrogen chloride gas was charged at a rate of 20 kg/hr to be aged for 1 hour. After the termination of the reaction, the conversion rate of the xylylene diamine was obtained by a neutralization titration method, so that the amine conversion rate was 99.80 mol%. The viscosity of the obtained reaction liquid (hydrochloride slurry) measured at 120°C by using an LVT viscometer manufactured by BROOKFIELD was 201 mPa·s. The reaction liquid had sufficient flowability. When the average particle size (number average value) of the hydrochloride particles was measured in an acetonitrile solvent with a laser diffraction particle size distribution measuring device: SALD-2100 manufactured by Shimadzu Corporation, the average particle size (number average value) of the hydrochloride particles was 25 µm. It was confirmed that the obtained hydrochloride slurry was liquid and had excellent flowability, and when the hydrochloride was transferred to the next step, the hydrochloride did not remain inside of the reaction vessel, and the liquid transfer properties were excellent.

Next, in the reaction vessel, after the temperature of the reaction liquid (hydrochloride slurry) was increased to 160°C, the phosgene was introduced at a rate of 100 kg/hr (1.0 kmol/hr) through the phosgene introduction tube to react for 8 hours, while the temperature thereof was retained. After the termination of the reaction, the nitrogen was purged in the reaction vessel, so that the unreacted phosgene and the hydrogen chloride gas were removed. Then, the reaction liquid was filtrated, so that 0.8 kg (dry weight) of the unreacted hydrochloride was removed. The obtained filtrate was desolvated, so that 188.6 kg (purity conversion yield of 98.30 mol%) of m-xylylene diisocyanate having a purity of 98.10% and containing 0.1 mass% of methachloromethylbenzyl isocyanate (hereinafter, may be referred to as CBI) was obtained. The hydrochloride conversion rate of the hydrochloride of the xylylene diamine at this time was 99.62%.

Next, by using a rectifier that was filled with a regular filling in a glass tower having an inner diameter of 50 mm and a length of 2000 mm, the obtained m-xylylene diisocyanate was refined. The m-xylylene diisocyanate was heated at 155°C with a preheater and supplied from the central portion of the tower at a rate of 1000 g/hr. The operating pressure was set at 0.20 kPa at the tower top, and as the operating temperature, the reboiler temperature was set at 150°C. At this time, the tower top temperature was 130°C. The rectifier was continuously operated for 20 hours, while each of 1000 g of m-xylylene diisocyanate was distilled off at a tower top reflux ratio of 20. In the xylylene diisocyanate composition that was distilled off from the tower top after reaching a steady state, the purity of the m-xylylene diisocyanate was 99.99 mass%, and the methachloromethylbenzyl isocyanate was 100 mass ppm.

### (Preparation Examples 2 to 8)

By changing the tower top reflux ratio of the rectifier, a xylylene diisocyanate composition containing a methachloromethylbenzyl isocyanate having a predetermined amount was obtained.

The content ratio of the methachloromethylbenzyl isocyanate in the m-xylylene diisocyanate composition in each of Preparation Examples is shown in Table 1.

### [Table 1]

**Table 1**

| Preparation Ex. No. | Content Ratio of CBI in XDI Composition (ppm) |
|---|---|
| Preparation Ex. 1 | 100 |
| Preparation Ex. 2 | 200 |
| Preparation Ex. 3 | 400 |
| Preparation Ex. 4 | 500 |
| Preparation Ex. 5 | 0.1 |
| Preparation Ex. 6 | 700 |
| Preparation Ex. 7 | 32 |
| Preparation Ex. 8 | 1800 |

### <Description of Abbreviations in Table 1>

XDI: xylylene diisocyanate
CBI: chloromethylbenzyl isocyanate
3. Production of Resin (Plastic Lens)

### (Example 1)

In a flask that was sufficiently dried, 36.4 g of the xylylene diisocyanate composition obtained in Preparation Example 1 containing the m-xylylene diisocyanate and 100 mass ppm of the methachloromethylbenzyl isocyanate, 0.001 g of dibutyl tin dichloride, 0.07 g of ZELEC UN (trade name, alkyl acid phosphate, internal mold release agent, manufactured by Stepan Company), and 0.05 g of Biosorb 583 (trade name, ultraviolet absorber, manufactured by SAKAI CHEMICAL INDUSTRY CO., LTD.) were weighed and stirred at 25°C for 1 hour to be mixed and dissolved, thereby preparing a polyisocyanate component. Thereafter, 33.6 g of 1,2-bis[(2-mercaptoethyl)thio]-3-mercaptopropane was charged and mixed in the polyisocyanate component, thereby preparing a liquid mixture (polymerizable composition).

The air in the liquid mixture was released at 600 Pa for 1 hour to be then filtrated with a 3-µm PTFE filter. Thereafter, the obtained liquid mixture was injected in a mold made of a glass mold and a tape. The mold was put into an oven, the temperature thereof was gradually increased from 10°C to 120°C, and then, the liquid mixture was polymerized for 18 hours. After the termination of the polymerization, the mold was taken out from the oven, and the inside was released from the mold, thereby obtaining a resin (plastic lens). The obtained resin was further annealed at 130°C for 4 hours. The Y.I value of the obtained resin was 4.5.

### (Example 2)

A resin (plastic lens) was produced in the same manner as in Example 1, except that the xylylene diisocyanate composition obtained in Preparation Example 2 containing the m-xylylene diisocyanate and 200 mass ppm of the methachloromethylbenzyl isocyanate was used instead of the xylylene diisocyanate composition used in Example 1.

### (Example 3)

A resin (plastic lens) was produced in the same manner as in Example 1, except that the xylylene diisocyanate composition obtained in Preparation Example 3 containing the m-xylylene diisocyanate and 400 mass ppm of the methachloromethylbenzyl isocyanate was used instead of the xylylene diisocyanate composition used in Example 1.

### (Example 4)

A resin (plastic lens) was produced in the same manner as in Example 1, except that the xylylene diisocyanate composition obtained in Preparation Example 4 containing the m-xylylene diisocyanate and 500 mass ppm of the methachloromethylbenzyl isocyanate was used instead of the xylylene diisocyanate composition used in Example 1.

### (Example 5)

In a flask that was sufficiently dried, 50.7 g of the xylylene diisocyanate composition obtained in Preparation Example 1 containing the m-xylylene diisocyanate and 100 mass ppm of the methachloromethylbenzyl isocyanate, 0.01 g of dibutyl tin dichloride, 0.1 g of ZELEC UN (trade name, alkyl acid phosphate, internal mold release agent, manufactured by Stepan Company), and 0.05 g of Biosorb 583 (trade name, ultraviolet absorber, manufactured by SAKAI CHEMICAL INDUSTRY CO., LTD.) were weighed and stirred at 25°C for 1 hour to be mixed and dissolved, thereby preparing a polyisocyanate component. Thereafter, 49.3 g of polythiol mainly composed of 4,8-dimercaptomethyl-1,11-dimercapto-3,6,9-trithiaundecane was charged and mixed in the polyisocyanate component, thereby preparing a liquid mixture (polymerizable composition).

The air in the liquid mixture was released at 600 Pa for 1 hour to be then filtrated with a 3-µm PTFE filter. Thereafter, the obtained liquid mixture was injected in a mold made of a glass mold and a tape. The mold was put into an oven, the temperature thereof was gradually increased from 10°C to 120°C, and then, the liquid mixture was polymerized for 18 hours. After the termination of the polymerization, the mold was taken out from the oven, and the inside was released from the mold, thereby obtaining a resin (plastic lens). The obtained resin was further annealed at 130°C for 4 hours. The Y.I value of the obtained resin was 4.5.

### (Example 6)

A resin (plastic lens) was produced in the same manner as in Example 5, except that the xylylene diisocyanate composition obtained in Preparation Example 2 containing the m-xylylene diisocyanate and 200 mass ppm of the methachloromethylbenzyl isocyanate was used instead of the xylylene diisocyanate composition used in Example 5.

### (Example 7)

A resin (plastic lens) was produced in the same manner as in Example 5, except that the xylylene diisocyanate composition obtained in Preparation Example 3 containing the m-xylylene diisocyanate and 400 mass ppm of the methachloromethylbenzyl isocyanate was used instead of the xylylene diisocyanate composition used in Example 5.

### (Example 8)

A resin (plastic lens) was produced in the same manner as in Example 5, except that the xylylene diisocyanate composition obtained in Preparation Example 4 containing the m-xylylene diisocyanate and 500 mass ppm of the methachloromethylbenzyl isocyanate was used instead of the xylylene diisocyanate composition used in Example 5.

### (Comparative Example 1)

Although 36.4 g of the xylylene diisocyanate composition obtained in Preparation Example 5 containing the m-xylylene diisocyanate and 0.1 mass ppm of the methachloromethylbenzyl isocyanate, 0.001 g of dibutyl tin dichloride, 0.07 g of ZELEC UN (trade name, alkyl acid phosphate, internal mold release agent, manufactured by Stepan Company), and 0.05 g of Biosorb 583 (trade name, ultraviolet absorber, manufactured by SAKAI CHEMICAL INDUSTRY CO., LTD.) were weighed and stirred at 25°C for 1 hour, the resulting mixture was not dissolved, so that a resin (plastic lens) could not be produced.

### (Comparative Example 2)

A resin (plastic lens) was produced in the same manner as in Example 1, except that the xylylene diisocyanate composition obtained in Preparation Example 6 containing the m-xylylene diisocyanate and 700 mass ppm of the methachloromethylbenzyl isocyanate was used instead of the xylylene diisocyanate composition used in Example 1.

### (Comparative Example 3)

Although 5.7 g of the xylylene diisocyanate composition obtained in Preparation Example 5 containing the m-xylylene diisocyanate and 0.1 mass ppm of the methachloromethylbenzyl isocyanate, 0.001 g of dibutyl tin dichloride, 0.07 g of ZELEC UN (trade name, alkyl acid phosphate, internal mold release agent, manufactured by Stepan Company), and 0.05 g of Biosorb 583 (trade name, ultraviolet absorber, manufactured by SAKAI CHEMICAL INDUSTRY CO., LTD.) were weighed and stirred at 25°C for 1 hour, the resulting mixture was not dissolved, so that a resin (plastic lens) could not be produced.

### (Comparative Example 4)

A resin (plastic lens) was produced in the same manner as in Example 5, except that the xylylene diisocyanate composition obtained in Preparation Example 6 containing the m-xylylene diisocyanate and 700 mass ppm of the methachloromethylbenzyl isocyanate was used instead of the xylylene diisocyanate composition used in Example 5.

The evaluation result of the Y.I value of each of the resins (plastic lenses) obtained in Examples 1 to 8 and Comparative Examples 2 and 4 is shown in Table 2.

### [Table 2]

**Table 2**

| | Content Ratio of CBI in XDI Composition (ppm) | Y.I Value of Resin |
|---|---|---|
| Ex. 1 | 100 | 4.5 |
| Ex. 2 | 200 | 4.5 |
| Ex. 3 | 400 | 4.5 |
| Ex. 4 | 500 | 4.5 |
| Ex. 5 | 100 | 4.5 |
| Ex. 6 | 200 | 4.5 |
| Ex. 7 | 400 | 4.5 |
| Ex. 8 | 500 | 4.5 |
| Comp. Ex. 2 | 700 | 4.7 |
| Comp. Ex. 4 | 700 | 4.8 |

### <Description of Abbreviations in Table 2>

XDI: xylylene diisocyanate
CBI: chloromethylbenzyl isocyanate

### (Example 9)

In a flask that was sufficiently dried, 35.5 g of the xylylene diisocyanate composition obtained in Preparation Example 7 containing the m-xylylene diisocyanate and 32 mass ppm of the methachloromethylbenzyl isocyanate, 0.007 g of dibutyl tin dichloride, 0.07 g of ZELEC UN (trade name, alkyl acid phosphate, internal mold release agent, manufactured by Stepan Company), and 0.035 g of Biosorb 583 (trade name, ultraviolet absorber, manufactured by SAKAI CHEMICAL INDUSTRY CO., LTD.) were weighed and stirred at 25°C for 1 hour to be mixed and dissolved, thereby preparing a polyisocyanate component. Thereafter, 34.5 g of mixture of 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane was charged and mixed in the polyisocyanate component, thereby preparing a liquid mixture (polymerizable composition).

The air in the liquid mixture was released at 600 Pa for 1 hour to be then filtrated with a 3-µm PTFE filter. Thereafter, the obtained liquid mixture was injected in a mold made of a glass mold and a tape. The mold was put into an oven, the temperature thereof was gradually increased from 10°C to 120°C, and then, the liquid mixture was polymerized for 18 hours. After the termination of the polymerization, the mold was taken out from the oven, and the inside was released from the mold, thereby obtaining a resin (plastic lens). The obtained resin was further annealed at 130°C for 4 hours.

### (Example 10)

A resin (plastic lens) was produced in the same manner as in Example 9, except that the xylylene diisocyanate composition obtained in Preparation Example 2 containing the m-xylylene diisocyanate and 200 mass ppm of the methachloromethylbenzyl isocyanate was used instead of the xylylene diisocyanate composition used in Example 9.

### (Comparative Example 5)

A resin (plastic lens) was produced in the same manner as in Example 9, except that the xylylene diisocyanate composition obtained in Preparation Example 8 containing the m-xylylene diisocyanate and 1800 mass ppm of the methachloromethylbenzyl isocyanate was used instead of the xylylene diisocyanate composition used in Example 9.

The evaluation result of the specific gravity, the dyeability (light transmittance), the optical properties (reflux index, Abbe number), and the thermal properties (heat resistance) of each of the resins (plastic lenses) obtained in Examples 9 and 10 and Comparative Example 5 is shown in Table 3.

### [Table 3]

**Table 3**

| Ex. No. | | Ex. 9 | Ex. 10 | Comp. Ex. 5 |
|---|---|---|---|---|
| Type of XDI Composition | | Preparation Ex. 7 | Preparation Ex. 2 | Preparation Ex. 8 |
| Content Ratio of CBI (ppm) | | 32 | 200 | 1800 |
| Specific Gravity of Resin at 20°C | | 1.370 | 1.370 | 1.370 |
| Optical Properties (546.1 nm) | Refractive Index (20°C) | 1.668 | 1.668 | 1.668 |
| | Abbe Number (20°C) | 31.0 | 31.6 | 31.6 |
| Dyeability (Light Transmittance) (%) | 638 nm | 61.4 | 62.0 | 64.1 |
| | 567 nm | 57.8 | 58.4 | 60.3 |
| | 452 nm | 56.0 | 56.9 | 58.5 |
| Thermal Properties | Heat Resistance (°C) | 104.1 | 104.3 | 103.9 |

### <Description of Abbreviations in Table 3>

XDI: xylylene diisocyanate
CBI: chloromethylbenzyl isocyanate

### Industrial Applicability

The xylylene diisocyanate composition and the polymerizable composition of the present invention are, for example, preferably used as various industrial materials such as polyurethane materials. The resin of the present invention is, for example, preferably used as various industrial products such as coating materials (paints, adhesives), elastomers, foams, and optical materials.

## Claims

1. A xylylene diisocyanate composition comprising:
a xylylene diisocyanate and a chloromethylbenzyl isocyanate, wherein
the content ratio of the chloromethylbenzyl isocyanate is 10 mass ppm or more and 500 mass ppm or less.

2. Use of the xylylene diisocyanate composition according to claim 1 in the production of an optical material.

3. A resin being a reaction product of:
the xylylene diisocyanate composition according to claim 1 and
a polyol component and/or a polythiol component.

4. The resin according to claim 3 being an optical material.

5. The resin according to claim 4 being an optical lens.

6. A polymerizable composition comprising:
the xylylene diisocyanate composition according to claim 1 and
a polyol component and/or a polythiol component.

## Patentansprüche

1. Xylylendiisocyanatzusammensetzung, umfassend:
ein Xylylendiisocyanat und ein Chlormethylbenzylisocyanat, wobei
das Gehaltsverhältnis des Chlormethylbenzylisocyanats 10 Massen-ppm oder mehr und 500 Massen-ppm oder weniger beträgt.

2. Verwendung der Xylylendiisocyanatzusammensetzung gemäß Anspruch 1 bei der Herstellung eines optischen Materials.

3. Harz, das ein Reaktionsprodukt ist von:
der Xylylendiisocyanatzusammensetzung gemäß Anspruch 1 und
einer Polyolkomponente und/oder einer Polythiolkomponente.

4. Harz gemäß Anspruch 3, das ein optisches Material ist.

5. Harz gemäß Anspruch 4, das eine optische Linse ist.

6. Polymerisierbare Zusammensetzung, umfassend:
die Xylylendiisocyanatzusammensetzung gemäß Anspruch 1 und
eine Polyolkomponente und/oder eine Polythiolkomponente.

## Revendications

1. Composition de xylylène diisocyanate comprenant :
du xylylène diisocyanate et du chlorométhylbenzyle isocyanate, dans laquelle
le rapport de teneur du chlorométhylbenzyle isocyanate est de 10 ppm en masse ou plus et de 500 ppm en masse ou moins.

2. Utilisation de la composition de xylylène diisocyanate selon la revendication 1 dans la production d'un matériau optique.

3. Résine qui est un produit de réaction :
de la composition de xylylène diisocyanate selon la revendication 1 et
d'un composant polyol et/ou d'un composant polythiol.

4. Résine selon la revendication 3 qui est un matériau optique.

5. Résine selon la revendication 4 qui est une lentille optique.

6. Composition polymérisable comprenant :
la composition de xylylène diisocyanate selon la revendication 1 et
un composant polyol et/ou un composant polythiol.
